# EUROPÄISCHE PATENTSCHRIFT

(11) **EP 1 594 890 B1**
(45) Veröffentlichungstag und Bekanntmachung des Hinweises auf die Patenterteilung: **11.04.2007**
(21) Anmeldenummer: 04708760.6
(22) Anmeldetag: 06.02.2004
(51) Int. Cl.: C07K 14/05, G01N 33/569

(54) **VON VCA-P18 KAPSIDANTIGEN DES EPSTEIN-BARR-VIRUS ABGELEITETE PEPTIDE UND IHRE VERWENDUNG**
PEPTIDE DERIVED FROM A VCA-P18 CAPSIDE ANTIGEN OF THE EPSTEIN-BARR VIRUS AND THE USE THEREOF
PEPTIDE DERIVE DE L'ANTIGENE DE CAPSIDE VCA-P18 DU VIRUS D'EPSTEIN-BARR ET SON UTILISATION

(30) Priorität: 21.02.2003 DE 10307517
(43) Veröffentlichungstag der Anmeldung: 16.11.2005
(73) Patentinhaber: Mikrogen Molekularbiologische Entwicklungs-GmbH, 82061 Neuried (DE)
(72) Erfinder: MOTZ, Manfred, 80689 München (DE); BAUER, Georg, 79104 Freiburg (DE); SOUTSCHEK, Erwin, 82335 Berg (DE)
(74) Vertreter: Keller, Günter
(86) Internationale Anmeldenummer: PCT/EP2004/001127
(87) Internationale Veröffentlichungsnummer: WO 2004/074317

(56) Entgegenhaltungen:
- US-A1- 2002 169 286
- HINDERER W ET AL: "Serodiagnosis of Epstein-Barr virus infection by using recombinant viral capsid antigen fragments and autologous gene fusion." JOURNAL OF CLINICAL MICROBIOLOGY. UNITED STATES OCT 1999, Bd. 37, Nr. 10, Oktober 1999 (1999-10), Seiten 3239-3244, XP002282389 ISSN: 0095-1137
- TRANCHAND-BUNEL D ET AL: "Evaluation of an Epstein-Barr virus (EBV) immunoglobulin M enzyme-linked immunosorbent assay using a synthetic convergent peptide library, or mixotope, for diagnosis of primary EBV infection." JOURNAL OF CLINICAL MICROBIOLOGY. UNITED STATES JUL 1999, Bd. 37, Nr. 7, Juli 1999 (1999-07), Seiten 2366-2368, XP002282390 ISSN: 0095-1137
- GRUNSVEN VAN W M J ET AL: "LOCALIZATION AND DIAGNOSTIC APPLICATION OF IMMUNODOMINANT DOMAINS OF THE BFRF3-ENCODED EPSTEIN-BARR VIRUS CAPSID PROTEIN" JOURNAL OF INFECTIOUS DISEASES, CHICAGO, IL, US, Bd. 170, 1. Juli 1994 (1994-07-01), Seiten 13-19, XP000619229 ISSN: 0022-1899
- BAUER G: "SIMPLICITY THROUGH COMPLEXITY: INNUNOBLOT WITH RECOMBINANT ANTIGENS AS THE NEW GOLD STANDARD IN EPSTEIN-BARR VIRUS SEROLOGY" CLINICAL LABORATORY, CLIN LAB PUBLICATIONS, HEIDELBERG, DE, Bd. 47, Nr. 5/6, 2001, Seiten 223-230, XP008021514 ISSN: 1433-6510

## Beschreibung

Die vorliegende Erfindung betrifft das Gebiet der Virusdiagnostik, insbesondere der Epstein-Barr-Virus (EBV)-Diagnostik. Es werden verbesserte Verfahren zum Nachweis von EBV-Infektionen und dazu geeignete Mittel bereitgestellt.

Das Epstein-Barr-Virus ist ein humanes Herpesvirus, das als Auslöser der infektiösen Mononukleose gilt. Die Infektion mit EBV verläuft oft subklinisch, gelegentlich mit leichten Symptomen. Bei immunsupprimierten Personen kann eine EBV-Infektion aber zu schweren malignen Manifestationen führen.

### Bedeutung der Epstein-Barr-Virus (EBV)-Diagnostik

Die EBV-Diagnostik besitzt eine große differentialdiagnostische Bedeutung, da die Symptomatik der akuten EBV-Infektion mit vielen klinischen Bildern überlappen kann, die durch andere Erreger oder Ursachen hervorgerufen werden. So kann eine akute EBV-Infektion mit einer HIV-Primärinfektion, Rötelnvirusinfektion, Zytomegalievirusinfektion, Infektion mit klassischen Hepatitisviren, Toxoplasmose, Leptospirose, Infektion mit verschiedenen neurotropen Erregern und mit einer Leukämie oder einem Lymphom verwechselt werden. Eine eindeutige EBV-Serologie stellt damit eine große Verpflichtung gegenüber dem Patienten dar.

### Probleme der EBV-Serologie

Die klassische EBV-Serologie beruht auf dem Nachweis der IgG- und IgM-Antikörperantwort gegen virale Kapsidantigene (VCA) und gegen EBV-spezifische Kernantigene (EBNA), besonders EBNA-1.

Die Einordnung verschiedener EBV-Antigen-Klassen beruht auf dem biologischen Zyklus der Virusvermehrung mit früh (EA) und spät (VCA) benötigten Proteinen sowie Antigenen zur Aufrechterhaltung der Latenz (EBNA). Diese Antigenklassen waren in den ersten serologischen Testsystemen (Immunfluoreszenz mit EBV-infizierten Zellen) nachweisbar und unterscheidbar. Alle weiteren nachfolgenden Entwicklungen an serologischen Testsystemen behielten dieses Schema bei und erlauben somit die Unterscheidung von Antikörperspezifitäten gegen EBNA, VCA und EA.

Die Marker VCA und EBNA wurden zunächst in der lmmunfluoreszenztechnik definiert. In neueren Tests werden die gereinigten, meist rekombinant hergestellten Komponenten p18 und p23 des VCA-Komplexes und p72, das dem EBNA-1 entspricht, verwendet. Das EBNA-1 Antigen wird spät nach einer Erstinfektion mit der Etablierung latent infizierter Zellen gebildet und ist für die Aufrechterhaltung dieses Status mit verantwortlich. Das bedeutet, daß anti-EBNA-1 erst mit einer Verzögerung von bis zu sechs Monaten nach Erstinfektion gebildet wird. Gleichzeitig bedeutet es aber auch, daß bei Vorhandensein dieses Markers ein sicherer Ausschluß einer frischen Infektion diagnostiziert werden kann und damit ein wichtiger Marker für eine zeitliche Einordnung des Infektionszeitpunktes gegeben ist.

EBV-Serologie weist ein hohes Maß an Variabilität auf, diese führt häufig zu uneindeutigen oder falschen Befunden, wenn nur die klassischen Marker VCA-IgG, VCA-IgM und Anti-EBNA-1 bestimmt werden. So zeigen beispielsweise nicht alle akuten EBV-Infektionen eine nachweisbare VCA-IgM-Antwort, so daß hier Teststrategien, die nur auf der Bestimmung von VCA-IgG und VCA-IgM beruhen, zu einer falschen Schlußfolgerung führen. Andererseits kann eine VCA-IgM-Antwort in seltenen Fällen auch monatelang persistieren und dadurch eine frische Infektion vortäuschen.

Positives Anti-EBNA-1 beweist eine abgelaufene EBV-Infektion und stellt in Kombination mit positivem VCA-IgG die einzige eindeutige serologische Konstellation dar. Negatives Anti-EBNA-1 (bei gleichzeitig positivem VCA-IgG) kann entweder eine akute EBV-Infektion anzeigen oder durch sekundären Anti-EBNA-1-Verlust bei Immunsuppression verursacht sein. Außerdem entwickeln rund 5 % der Infizierten niemals nachweisbares Anti-EBNA-1 und täuschen damit lebenslang die serologische Situation einer akuten EBV-Infektion vor.

So wird durch gleichzeitig positives VCA-IgG und Anti-EBNA-1 eine akute EBV-Infektion sicher ausgeschlossen. Positives VCA-IgG bei negativem Anti-EBNA-1 stellt jedoch eine uneindeutige Konstellation dar, die in drei Situationen auftritt:
1) bei frischer EBV-Infektion;
2) bei abgelaufener Infektion und Anti-EBNA-1-Verlust durch Suppression des zellulären Immunsystems;
3) bei abgelaufener Infektion und gleichzeitig fehlender Anti-EBNA-1-Bildung (bei rund 5% der gesunden Bevölkerung).

Die Fälle 2) und 3) sind z. B. im Untersuchungsgut großer Kliniken häufiger zu finden als die echte frische EBV-Infektion.

Dieses zentrale Problem der EBV-Serologie läßt sich bisher nur durch aufwendige, zusätzliche Tests oder durch Wiederholungen im zeitlichen Verlauf im Zusammenhang mit klinischen Daten verläßlich lösen.

Das Fehlen von anti-EBNA-1 wird deshalb in allgemeinen als Indikator einer Frischinfektion verwendet werden. Dies kann - wie oben angeführt - zu drastischen Fehlinterpretationen im Falle von EBV-infizierten Personen führen; die anti-EBNA-1-negativ bleiben oder aufgrund Suppression des zellulären Immunsystems Antikörper dagegen verlieren. Die derzeitigen, in der Routine-Diagnostik verwendeten Verfahren mit anti-EBNA-1 erlauben nicht mit Sicherheit die Diagnose einer frischen EBV-Infektion.

Die Abgrenzung primärer Anti-EBNA-1-Negativität bei akuter EBV-Infektion einerseits, von Anti-EBNA-1-Verlust und fehlender Anti-EBNA-1-Bildung andererseits, stellt somit das größte Problem der EBV-Serologie dar. Diese besondere Problematik wird bei den meisten publizierten Evaluationen serologischer Tests nicht berücksichtigt. Es muß angenommen werden, daß bis zu 20 Prozent der Untersuchungen auf EBV-Infektion uneindeutig sind und zu Fehlbefunden führen. Diese sind besonders in der Onkologie, der Transplantationsmedizin und bei der Betreuung in der Schwangerschaft von gravierender Bedeutung.

Hinderer W. et al., Journal of Clinical Microbiology, United States, Oct. 1999, Bd. 37, Nr. 10, Oktober 1999 (1999-10), Seiten 3239-3244, offenbaren ein GST-p18 EBV Fusionskonstrukt, welches p18 105-176 enthält. Dieses Konstrukt erkennt bezüglich der IgG Antikörper besser zurückliegende Infektionen als Primärinfektionen und reagiert auch weniger gut mit Antikörpern aus Primärinfektionen im Vergleich zu zurückliegenden Infektionen.

Bauer (Clin Lab 2001, 47, 223-230) beschreibt in einem Übersichtsartikel Immunoblots mit rekombinanten Antigenen bei der Epstein-Barr-Virus-Serologie.

Tranchand-Bunel et al., Journal of Clinical Microbiology 1999, 2399-2368, erläutern IgM ELISA-Tests, bei denen Peptide des VCA p18 Kapsid-Antigens eingesetzt werden.

van Grunsven et al. (Journal of Infectious Diseases, 1994, 13-19) beschreiben immundominante Epitope am C-Terminus des VCA p18 Kapsid-Proteins.

Eine Aufgabe der vorliegenden Erfindung ist es, Mittel zum Nachweis einer EBV-Infektion bereitzustellen, die eine sicherere Unterscheidung von akuter und abgelaufener Infektion ermöglichen.

Überraschenderweise wurde gefunden, daß durch Modifikation des viralen EBV-Kapsidantigens p18 ein Peptid erhalten werden kann, das mit Antikörpern reagiert, die mit einer ähnlichen Sicherheit wie anti-EBNA-1 erst spät nach Infektion gebildet werden und die Problematik der Nicht-Bildung oder das Verschwinden bei Immunsupprimierten nicht im selben Umfang zeigen.

Es handelt sich dabei um Peptide, die von dem EBV-p18 Antigen abgeleitet sind, welches vom Leserahmen BFRF3 codiert wird. Dieses Antigen wurde schon 1988 als VCA-Marker beschrieben (Middeldorp und Herbrink, J. Virol. Meth., 21 (1988) 133 - 146).

Die molekulare Zuordnung von p18 und die Darstellung des Proteins mittels gentechnologischer Methoden sowie die Verwendung bestimmter immunologisch aktiver Fragmente daraus sind in EP 574 075 A2 beschrieben. Allerdings wurde bei der Beschreibung der Eigenschaften der rekombinanten Antigene bzw. der chemisch synthetisierten Fragmente immer auf eine optimale Reaktivität abgestellt.

lgG-Antikörper gegen ein rekombinant hergestelltes, gereinigtes komplettes p18-Antigen sind schon bei frischer EBV-Infektion nachweisbar und eignen sich daher nicht zur Diskriminierung zwischen akuten und abgelaufenen Infektionen.

Überraschenderweise hat sich im Verlauf von Untersuchungen gezeigt, daß beispielsweise durch das Weglassen des N-terminalen Bereichs des p18-Antigens eine zeitliche Diskriminierung von Antikörper-Antworten erreicht wird: N-terminal deletiertes p18 erkennt Antikörper, die erst Wochen nach Infektion gebildet werden.

Die vorliegende Erfindung betrifft daher ein Peptid, das vom viralen p18-Antigen des Epstein-Barr-Virus abgeleitet ist und dadurch gekennzeichnet ist, daß es mit Antikörpern aus Individuen mit länger zurückliegender Epstein-Barr-Virus-Infektion reagiert, aber nicht oder schwach mit Antikörpern aus Individuen mit akuter oder kurz zurückliegender Epstein-Barr-Virus-Infektion, dadurch gekennzeichnet, dass es gegenüber dem viralen p18 Antigen N-terminal um 10 - 70 Aminosäuren verkürzt ist.

Der Ausdruck "Peptid" umfaßt Oligopeptide und Polypeptide. Die Länge der Peptide ist nicht beschränkt, soweit es nicht angegeben ist.

Das erfindungsgemäße Peptid ist ein modifiziertes p18-Antigen, d.h. es ist nicht identisch mit dem viralen p18, dem "Wildtyp-Protein". Die Modifikationen gegenüber dem viralen p18 sind nicht besonders eingeschränkt, solange das modifizierte Peptid mit Antikörpern aus Individuen mit länger zurückliegender EBV-Infektion reagiert, aber nicht oder schwach mit Antikörpern aus Individuen mit akuter oder kurz zurückliegender EBV-Infektion und, das Antigen ist dadurch gekennzeichnet, dass es gegenüber dem viralen p18 Antigen N-terminal um 10 - 70 Aminosäuren verkürzt ist.

Daß das erfindungsgemäße Peptid von p18 abgeleitet ist, bedeutet, daß es wenigstens 1 Fragment mit wenigstens 1 Epitop des vollständigen viralen p18-Antigens umfaßt. Ein solches Epitop ist vorhanden, wenn in einem line assay (siehe Beispiel 3) bei Inkubation mit Seren aus Individuen mit länger zurückliegender EBV-Infektion eine positive Reaktion erhalten wird.

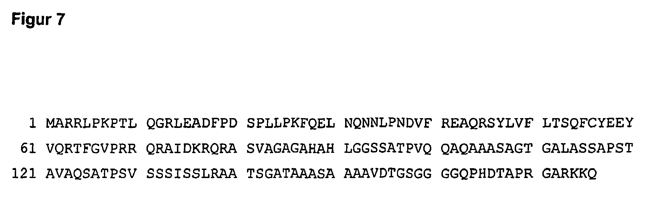

Die Aminosäuresequenz des viralen p18 ist in Figur 7 dargestellt (SEQ ID NO:1). In der Regel hat das erfindungsgemäße Peptid eine von SEQ ID NO:1 abweichende Aminosäuresequenz. Dabei handelt es sich vorzugsweise um eine oder mehrere Deletionen und/oder Substitutionen. Das erfindungsgemäße Peptid weist eine oder mehrere Deletionen gegenüber der in SEQ ID NO:1 dargestellten Aminosäuresequenz auf, so dass am N-Terminus von p18 deletiert 10-70 Aminosäuren sind.

Das erfindungsgemäße Peptid umfaßt oder besteht vorzugsweise aus wenigstens 125 aufeinanderfolgenden Aminosäuren der Aminosäuresequenz SEQ ID NO:1.

Es ist bevorzugt, daß in dem erfindungsgemäßen Peptid wenigstens ein Epitop des vollständigen p18-VCA, gegen das nach Infektion Antikörper gebildet werden, nicht vorhanden ist.

Das erfindungsgemäße Peptid ist gegenüber dem viralen p18-Antigen N-terminal um 10 bis 70 Aminosäuren, bevorzugt um 15 bis 50 Aminosäuren, bevorzugter um 20 bis 40 Aminosäuren, und noch bevorzugter um ungefähr 30 Aminosäuren, verkürzt. In einer besonderen Ausführungsform besteht das erfindungsgemäße Peptid aus der Aminosäuresequenz SEQ ID NO:2. Diese Aminosäuresequenz entspricht den Aminosäuren 31 bis 176 von SEQ ID NO:1. In weiteren bevorzugten Ausführungsformen hat das erfindungsgemäße Peptid im wesentlichen eine Aminosäuresequenz, die ausgewählt ist aus der Gruppe bestehend aus
Aminosäuren 21 bis 176 von SEQ ID NO:1,
Aminosäuren 22 bis 176 von SEQ ID NO:1,
Aminosäuren 23 bis 176 von SEQ ID NO:1,
Aminosäuren 24 bis 176 von SEQ ID NO:1,
Aminosäuren 25 bis 176 von SEQ ID NO:1,
Aminosäuren 26 bis 176 von SEQ ID NO:1,
Aminosäuren 27 bis 176 von SEQ ID NO:1,
Aminosäuren 28 bis 176 von SEQ ID NO:1,
Aminosäuren 29 bis 176 von SEQ ID NO:1,
Aminosäuren 30 bis 176 von SEQ ID NO:1,
Aminosäuren 31 bis 176 von SEQ ID NO:1,
Aminosäuren 32 bis 176 von SEQ ID NO:1,
Aminosäuren 33 bis 176 von SEQ ID NO:1,
Aminosäuren 34 bis 176 von SEQ ID NO:1,
Aminosäuren 35 bis 176 von SEQ ID NO:1,
Aminosäuren 36 bis 176 von SEQ ID NO:1,
Aminosäuren 37 bis 176 von SEQ ID NO:1,
Aminosäuren 38 bis 176 von SEQ ID NO:1,
Aminosäuren 39 bis 176 von SEQ ID NO:1,
Aminosäuren 40 bis 176 von SEQ ID NO:1 und
Aminosäuren 41 bis 176 von SEQ ID NO:1.

Dem Fachmann ist auch bewußt, daß die immunologischen Eigenschaften der angegebenen Sequenzen oft nur unwesentlich verändert werden, wenn Aminosäuren inseriert, substituiert oder deletiert sind. Substitutionen, die oft als konservativ angesehen werden, sind solche, bei denen die chemische Natur der substituierenden Aminosäure ähnlich der der substituierten Aminosäure ist. Aminosäurekombinationen, die als konservativ angesehen werden können, sind beispielsweise Gly/Ala, Asp/Glu, Asn/Gln, Val/Ile/Leu, Ser/Thr, Lys/Arg und Phe/Tyr.

Es können zusätzliche Aminosäuren oder chemische Gruppen am N- oder C-Terminus hinzugefügt werden, um eine Art "linker" zu schaffen, durch den das Peptid vorteilhaft an einen Träger gekoppelt werden kann. Ein solcher "linker" hat in der Regel 1 bis 60 Aminosäuren, meistens jedoch 1 bis 10 Aminosäuren. Die Bindung des Peptids an einen Träger oder eine feste Phase muß aber nicht kovalent sein. Es können ebenfalls Cysteinreste angefügt werden, um eine Kopplung mit anderen Peptiden zu erreichen.

Die erfindungsgemäßen Peptide können weiter modifiziert sein, beispielsweise durch Glycosylierung, Amidierung, Carboxylierung oder Phosphorylierung. Funktionelle Derivate wie z.B. Salze, Amide, Ester, C-amidierte oder N-acylierte Derivate sind von der Erfindung ebenfalls umfaßt.

Der Fachmann kann aufgrund der Informationen in dieser Anmeldung auf einfache Weise ermitteln, ob ein bestimmtes p18-Derivat geeignet ist, um zwischen akuter und abgelaufener Infektion zu unterscheiden. Dazu muß ein gegebenes p18-Derivat beispielsweise lediglich in einem line assay wie er in Beispiel 3 dargestellt ist, getestet werden.

Die erfindungsgemäßen Peptide können auf verschiedene, dem Fachmann bekannte Weisen hergestellt werden. In der Regel werden sie entweder durch chemische Synthese oder durch Expression rekombinanter DNA in geeigneten Wirtszellen hergestellt.

Als Verfahren zur chemischen Synthese von Peptiden kann Festphasensynthese oder Flüssigphasensynthese verwendet werden. Festphasensynthese ist bevorzugt. Verfahren zur chemischen Synthese von Peptiden sind beschrieben in Bodanszky und Ondetti: Peptide Synthesis, Interscience Publishers, New York (1966); The Peptides, Analysis, Synthesis, Biology Volume 1-3 (Ed. Gross und Maienhofer) 1979, 1980, 1981 (Academic Press, Inc.).

Zur rekombinanten Herstellung des erfindungsgemäßen Peptids wird zunächst eine Nukleinsäure, die für die Aminosäuresequenz des Peptides codiert, bereitgestellt. Die Nukleinsäure kann DNA oder RNA sein, DNA ist bevorzugt. Die codierende Nukleinsäure wird üblicherweise in einen Vektor oder ein Plasmid eingefügt, das geeignete Sequenzen enthält, die die Expression des codierten Peptids erlauben. Derartige geeignete Sequenzen sind beispielsweise ein Promotor, eine Terminatorsequenz und Sequenzen, die die Replikation des Plasmids ermöglichen. Es kann sich um Expressionsplasmide zur Expression in Prokaryonten oder in Eukaryonten handeln. Dem Fachmann ist bewußt, daß dabei unterschiedliche Promotorsequenzen verwendet werden. Prokaryontische Expressionsplasmide sind bevorzugt.

Die Erfindung betrifft auch einen Vektor oder ein Plasmid, das eine Nukleinsäure enthält, die für ein erfindungsgemäßes Peptid codiert. Übliche Verfahren zur Herstellung von Vektoren und Plasmiden sind beschrieben in Sambrook et al., Molecular Cloning: A Laboratory Manual, 2001, Cold Spring Harbour Laboratory Press. Weiterhin wird der Vektor oder das Plasmid in geeignete Wirtszellen, z.B. Säugerzellen oder bevorzugt Bakterien, eingebracht, der dann unter geeigneten Bedingungen kultiviert wird, so daß eine Expression des gewünschten Peptids erfolgt. Die Erfindung betrifft auch eine Zelle enthaltend einen erfindungsgemäßen Vektor oder ein erfindungsgemäßes Plasmid. Ein weiterer Aspekt der Erfindung ist ein Verfahren zur Herstellung eines erfindungsgemäßen Peptids, das umfaßt, daß man erfindungsgemäße Zellen unter geeigneten Bedingungen kultiviert, so daß des gewünschte Peptid exprimiert wird, und gegebenenfalls das Peptid gewinnt. Nach erfolgter Expression kann das Peptid durch dem Fachmann an sich bekannte Methoden gewonnen werden. Geeignete Verfahren zur rekombinanten Expression von Peptiden sind in Molecular Cloning: A Laboratory Manual von Sambrook et al. (siehe oben) beschrieben.

Vorzugsweise liegt das Peptid in isolierter Form vor, das heißt es ist im wesentlichen frei von anderen Peptiden. Die Reinheit des erfindungsgemäßen Peptids ist vorzugsweise größer 90%, bevorzugter größer 95%, am bevorzugtesten größer 99%, bestimmt anhand von SDS-PAGE mit anschließender Coomassiefärbung.

Daher wird das Peptid nach erfolgter Expression in der Regel gereinigt. Dabei können verschiedenste dem Fachmann bekannte Reinigungsverfahren eingesetzt werden. Verfahren zur Reinigung von Proteinen und Peptiden sind beschrieben in Methods in Enzymology, Vol. 182, Guide to Protein Purification, Academic Press, New York 1990 oder Scopes R.K., Protein Purification, Springer-Verlag, Heidelberg 1994.

Das erfindungsgemäße Peptid zeichnet sich dadurch aus, daß es mit Antikörpern aus Individuen mit länger zurückliegender Epstein-Barr Virusinfektion reagiert, aber nicht oder schwach mit Antikörpern aus Individuen mit akuter oder kurz zurückliegender Epstein-Barr Virusinfektion. Vorzugsweise reagiert das Peptid nicht mit Antikörpern aus Individuen mit kurz zurückliegender EBV-Infektion. Vorzugsweise beziehen sich die Angaben auf IgG-Antikörper.

Seren aus einem Individuum mit länger zurückliegender Infektion können folgende serologische Eigenschaften aufweisen:
- anti-EBNA-1-lgG: positiv;
- anti-p23-lgG: positiv;
- anti-p54-lgG: negativ;
- anti-p138-lgG: negativ; und
- anti-p23-IgM: negativ.

Das erfindungsgemäße Peptid reagiert mit Antikörper aus Seren, die diese serologischen Eigenschaften aufweisen.

In der Regel kann eine ungefähr 1,5 Jahre zurückliegende Infektion als "länger zurückliegende Infektion" bezeichnet werden.

Die Infektion eines Individuums mit EBV gilt als "akut", wenn das klinische Bild einer infektiösen Mononukleose vorliegt. Seren aus einem Individuum mit kurz zurückliegender EBV-Infektion können folgende serologische Eigenschaften aufweisen:
- anti-EBNA-1-lgG: negativ;
- anti-p54-IgG und/oder anti-p138-lgG (EA): positiv;
- anti-p23-lgG (VCA): positiv; und
- anti-p54- und/oder -p138- und/oder -p54-lgM: positiv.

Das erfindungsgemäße Peptid reagiert nicht oder schwach mit Antikörpern aus Seren, die diese serologischen Eigenschaften aufweisen.

In der Regel kann eine ungefähr 4 Wochen zurückliegende Infektion als "kurz zurückliegende Infektion" bezeichnet werden.

Der Ausdruck, daß ein Peptid mit Antikörpern "reagiert", bedeutet im Sinne der vorliegenden Anmeldung, daß das Peptid mit einem oder mehreren Antikörpern Antigen-Antikörper-Immunkomplexe bildet, nachdem Peptid und Antikörper miteinander in Kontakt gebracht worden sind. Keine oder eine schwache Reaktion des Peptids mit Antikörpern liegt vor, wenn keine oder nur schwache Antigen-Antikörper-Komplexe gebildet werden, nachdem Peptid und Antikörper miteinander in Kontakt gebracht worden sind. Ob ein Peptid mit Antikörpern reagiert, kann durch einen "line-assay" festgestellt werden, wie er in Beispiel 3 dargestellt ist.

Die Erfindung betrifft weiterhin ein Fusionspeptid, das ein oben beschriebenes erfindungsgemäßes Peptid umfaßt und eine oder mehrere weitere Aminosäuren, die nicht der Aminosäuresequenz des viralen p18-Antigens entsprechen. Dabei kann es sich um verschiedenste Fremdsequenzen handeln. Bevorzugt ist es aber, daß nur wenige Fremdaminosäuren in dem Fusionspeptid enthalten sind, um eine Kreuzreaktion der Fremdaminosäuren mit Antikörpern zu vermeiden. Beispiele für Fremdsequenzen sind Aminosäuresequenzen, die die Affinitätsreinigung des exprimierten Peptids erleichtern, z.B. ein Oligohistidin-stretch (6 bis 8 aufeinanderfolgende Histidinreste) am N- oder C-Terminus.

Ein weiterer Aspekt der Erfindung ist ein diagnostisches Reagenz zum Nachweis von Antikörpern gegen Epstein-Barr Virus, umfassend ein erfindungsgemäßes Peptid und/oder ein erfindungsgemäßes Fusionspeptid. Ein "diagnostisches Reagenz" im Sinne der vorliegenden Erfindung umfaßt eine feste Phase oder eine Nachweissubstanz. Feste Phasen können beispielsweise Mikrotestplatten, Küvetten, Gefäße, Membranen, Filter, Teststreifen oder Partikel wie Kügelchen sein. Verfahren zum Binden von Peptiden an feste Oberflächen oder feste Phasen sind dem Fachmann an sich bekannt.

Nachweissubstanzen können beispielsweise radioaktive Isotopen, fluoreszierende Verbindungen, Enzyme, Farbstoffe, etc. sein. Die erfindungsgemäßen Peptide oder Fusionspeptide können markiert oder unmarkiert sein, je nach der beabsichtigten Verwendung. Die Markierung kann jeder Art sein, z. B. enzymatisch, chemisch, fluoreszierend, lumineszierend oder radioaktiv.

Antikörper gegen Epstein-Barr Virus können beispielsweise nachgewiesen werden, indem ein diagnostisches Reagenz gemäß der vorliegenden Erfindung mit Serum, das von einem Individuum stammt, in Kontakt gebracht wird und anschließend die gebildeten Antigen-Antikörper-Komplexe nachgewiesen werden. Die Erfindung betrifft daher ebenfalls ein Verfahren zum Nachweis von Antikörpern gegen Epstein-Barr Virus in einer Probe, das umfaßt, daß man ein erfindungsgemäßes Peptid oder Fusionspeptid oder ein diagnostisches Reagenz gemäß der Erfindung mit der Probe in Kontakt bringt und anschließend die gebildeten Antigen-Antikörper-Komplexe nachweist.

Die Probe ist in der Regel eine Körperflüssigkeit, die von einem Individuum stammt. Vorzugsweise ist die Probe Serum, das von einem Individuum stammt. Das Serum kann mit einer geeigneten Flüssigkeit verdünnt werden.

Üblicherweise wird im erfindungsgemäßen Verfahren ein oben beschriebenes diagnostisches Reagenz eingesetzt. Abhängig von der Natur und weiteren Merkmalen des diagnostischen Reagenz ist die immunchemische Reaktion eine sogenannte Sandwich-Reaktion, eine Agglutinierungsreaktion, eine Kompetition oder eine Inhibition.

In einem bevorzugten Format des Verfahrens ist das erfindungsgemäße Peptid oder Fusionspeptid auf einer festen Phase immobilisiert. Die feste Phase wird dann mit der zu untersuchenden Probe, beispielsweise Serum in Kontakt gebracht, wodurch sich Antigen-Antikörperkomplexe bilden können. Die feste Phase wird anschließend ein oder mehrere Male gewaschen, um ungebundene Antikörper zu entfernen. Anschließend werden die immobilisierten Antigen-Antikörperkomplexe nachgewiesen. Dies kann beispielsweise durch sogenannte anti-Antikörper erfolgen, die an eine Markierungssubstanz gekoppelt sind. So kann nach dem Waschen die feste Phase mit einem anti-IgG-Antikörper, der an Meerrettichperoxidase gekoppelt ist, in Kontakt gebracht werden. Dieser anti-Antikörper bindet an die immobilisierten Immunkomplexe. Der gebundene sekundäre Antikörper kann durch die Enzymreaktion der Meerrettichperoxidase in bekannter Weise sichtbar gemacht werden. Der sekundäre Antikörper erkennt vorzugsweise spezifisch humanes IgG.

Vorzugsweise werden in dem Verfahren Membranen oder Teststreifen eingesetzt, auf denen ein oder mehrere Antigene immobilisiert sind. In einer anderen Ausführungsform des Verfahrens wird ein ELISA-Test durchgeführt. Die Durchführung derartiger Verfahren ist dem Fachmann an sich bekannt. Eine Übersicht über geeignete Nachweisverfahren findet sich in "Labor und Diagnose"; L. Thomas; Die medizinische Verlagsgesellschaft, Marburg; ISBN 3-921320-21-6.

Zur Durchführung eines ELISA wird üblicherweise ein Peptid oder eine Mischung mehrerer Peptide an eine feste Phase gebunden, z. B. an eine Mikrotiterplatte. Eine geeignete Verdünnung der zu testenden Körperflüssigkeit (Serum) wird mit der festen Phase, an die das oder die Peptide gebunden sind, in Kontakt gebracht. Die anschließende Inkubation wird für einen Zeitraum durchgeführt, der ausreichend ist zur Bildung von Antigen-Antikörper-Komplexen. Anschließend werden ungebundene Komponenten abgewaschen. Die Detektion der Immunkomplexe erfolgt in der Regel über Antikörper, die spezifisch an humane Immunglobuline binden und die markiert sind (geeignete Marker s.o.). Oft wird dann nach Zugabe eines Substrates ein Produkt gebildet, das leicht visuell, photometrisch, spektrometrisch, luminometrisch oder elektrochemisch nachgewiesen werden kann.

Eine andere Variante ist der Kompetitionsassay, der dem Fachmann auch bekannt ist.

In einer besonderen Ausführungsform des Verfahrens wird ein weiteres Antigen des Epstein-Barr Virus mit der Probe in Kontakt gebracht und anschließend die gebildeten Antigen-Antikörper-Komplexe nachgewiesen. Bei dem weiteren Antigen des EBV handelt es sich vorzugsweise um ein Antigen, das charakteristisch für eine frische Infektion ist. Vorzugsweise ist das weitere Antigen ausgewählt aus der Gruppe bestehend aus p18, p23, p138 und p54. In einer weiteren Ausführungsform des Verfahrens wird neben dem erfindungsgemäßen Peptid oder Fusionspeptid das späte nukleäre Antigen EBNA-1 (p72) eingesetzt. In einer weiteren Ausführungsform werden neben dem erfindungsgemäßen Peptid oder Fusionspeptid die Antigene p72, p18, p23, p138 und/oder p54 eingesetzt.

Ein weiterer Aspekt der Erfindung ist ein Testkit zum Nachweis von Antikörpern gegen Epstein-Barr Virus, der ein erfindungsgemäßes Peptid oder Fusionspeptid und/oder ein diagnostisches Reagenz, wie es oben beschrieben wurde, umfaßt. Der Testkit kann weitere, zur Durchführung des erfindungsgemäßen Verfahrens geeignete Mittel enthalten, beispielsweise eine Zusammensetzung, die einen sekundären Antikörper enthält. Vorzugsweise enthält der Testkit eine feste Phase. Besonders bevorzugt sind dabei Western Blot-Teststreifen, auf denen wenigstens ein erfindungsgemäßes Peptid oder Fusionspeptid immobilisiert ist. Es können weitere EBV-Antigene immobilisiert sein, z.B. p18, p23, p72, p54 und/oder p138. Dabei kann es sich um Nitrocellulosestreifen handeln. Der Testkit kann weiterhin Waschlösungen, gegebenenfalls in konzentrierter Form, umfassen.

Ein weiterer Aspekt der Erfindung ist die Verwendung eines modifizierten Viruskapsid-Antigens des Epstein-Barr Virus zur Bestimmung, ob eine akute/kurz zurückliegende oder eine länger zurückliegende/abgelaufene EBV-Infektion in einer Probe vorliegt. Die bevorzugten Ausführungsformen der erfindungsgemäßen Verwendung entsprechen den oben beschriebenen bevorzugten Ausführungsformen des Peptids, des Fusionspeptids, des diagnostischen Reagenz, des Testkits und/oder des Verfahrens. Die Erfindung betrifft weiterhin die Verwendung eines modifizierten Viruskapsid-Antigens des Epstein-Barr Virus zur serologischen Diskriminierung von frischen/kurz zurückliegenden und abgelaufenen Infektionen in einer Probe. Die Erfindung betrifft weiterhin die Verwendung eines modifizierten Viruskapsid-Antigens des Epstein-Barr Virus zum Nachweis von Antikörpern gegen EBV. Dabei kann in einer Probe vorzugsweise zwischen einer frischen und einer abgelaufenen Infektion unterschieden werden. Die Erfindung betrifft weiterhin die Verwendung eines modifizierten Viruskapsid-Antigens des Epstein-Barr Virus zum Nachweis einer abgelaufenen EBV-Infektion. Schließlich betrifft die Erfindung die Verwendung eines modifizierten Viruskapsid-Antigens des Epstein-Barr Virus zur Unterscheidung zwischen einer abgelaufenen und einer frischen EBV-Infektion in einer Probe. Das modifizierte Viruskapsid-Antigen gemäß den Verwendungen der Erfindung entspricht vorzugsweise dem erfindungsgemäßen Peptid oder Fusionspeptid. Alle weiter oben beschriebenen bevorzugten Varianten der Erfindung gelten sinngemäß für die Verwendungen der Erfindung.

Alle in der vorliegenden Anmeldung beschrieben Aspekte und bevorzugten Ausführungsformen der Erfindung können in verschiedenster Weise miteinander kombiniert werden. Derartige Kombinationen sind ebenfalls von der Erfindung erfaßt.

Durch die vorliegende Erfindung wird ein zweiter später Marker neben dem EBNA-1 bereitgestellt, der zudem nicht die Problematik der Nichtbildung von Antikörpern aufweist. Im Gegensatz zu EBNA-1 werden Antikörper gegen p18 nach zellulärer Immunsuppression nicht verloren. Der Nachweis von IgG gegen ein erfindungsgemäß modifiziertes p18 stellt demnach einen sicheren Ausschluß einer akuten EBV-Infektion dar.

Durch die Verwendung von modifiziertem p18 in geeigneten Meßsystemen, wie zum Beispiel dem lmmunblot, lassen sich also frische und abgelaufene EBV-Infektionen auch dann nachweisen, wenn die Anti-EBNA-Antwort einen untypischen Verlauf zeigt.

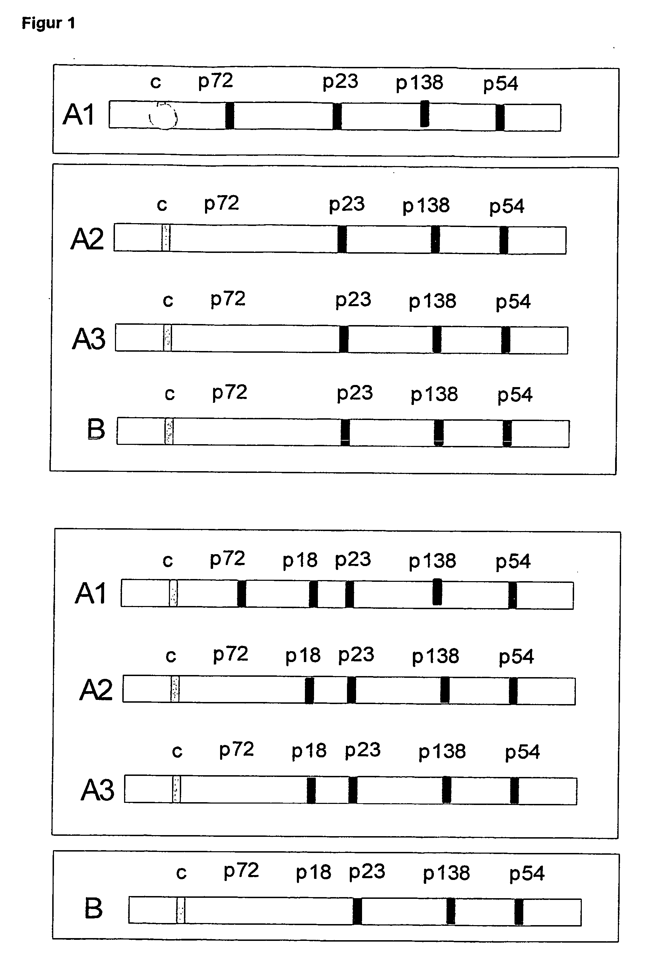

Figur 1 zeigt die Verbesserung der Aussagekraft der EBV-Diagnostik durch Verwendung eines modifizierten p18-Antigens.

Die Abbildung zeigt schematisch in der oberen Hälfte einen konventionellen Immunblot zur Bestimmung von Antikörpern gegen EBV und in der unteren Hälfte einen erfindungsgemäßen Test, der zusätzlich N-terminal verkürztes p18-Antigen beinhaltet (auf den Streifen als "p18" bezeichnet). Die Marker p23 (VCA), p54 (frühes Antigen) und p138 (frühes Antigen) stellen Seropositivitätsmarker dar, die nicht zwischen frischen und abgelaufenen Infektionen differenzieren können. Die Antikörperantwort gegen diese Marker ist variabel, so daß unterschiedliche Muster der Immunantwort erkennbar sein können. Positivität der Antikörperantwort gegen mindestens einen dieser Marker erlaubt die Diagnose "EBV-infiziert", ohne weitere Differenzierung zwischen "frischer Infektion" und "abgelaufener Infektion". Eine positive IgG-Antwort gegen p72 (= EBNA-1) erlaubt die sichere Diagnose "abgelaufene Infektion". Im konventionellen Test wird die klassische abgelaufene EBV-Infektion (A1) aufgrund des positiven p72-lgG richtig erkannt, während sich eine abgelaufene EBV-Infektion ohne p72-IgG-Bildung (A2) oder eine abgelaufene EBV-Infektion mit nachfolgendem sekundärem p72-lgG-Verlust (A3) nicht von einer echten frischen EBV-Infektion (B) mit noch nicht ausgebildeter p72-IgG-Antwort unterscheiden lassen. Die Fälle A2 und A3 werden also im konventionellen Test in der Regel falsch diagnostiziert. In der täglichen Praxis sind diese Fälle aber häufiger als die echten frischen EBV-Infektionen!

Bei Verwendung des modifizierten Tests (untere Hälfte), der z.B. N-terminal verkürztes p18 (in Figur 1 als "p18" bezeichnet) enthält, wird die frische EBV-Infektion mit fehlendem p18-IgG (B) klar von den abgelaufenen EBV-Infektionen (A1-A3) unterschieden, selbst dann, wenn p72-IgG fehlt.

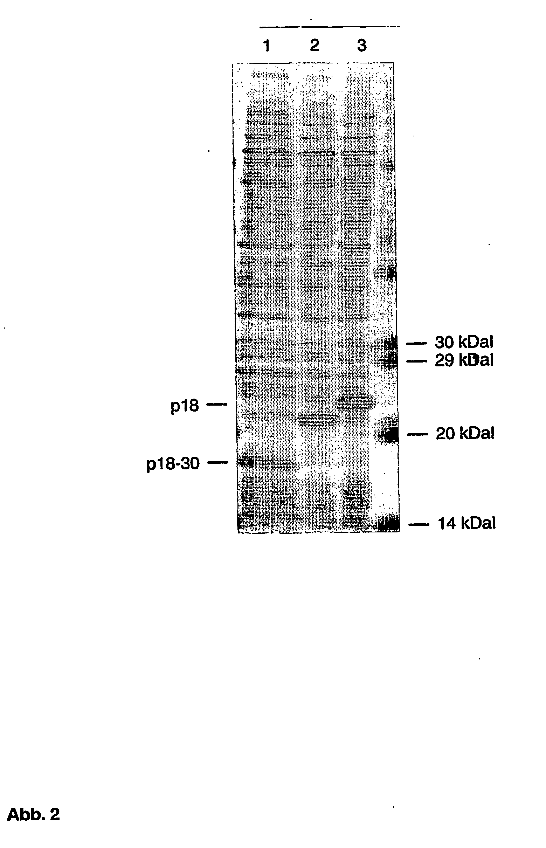

Figur 2 zeigt die Expression verschiedener Peptide. Es ist ein Coomassie-blau gefärbtes SDS-Polyacrylamid-Gel von Lysaten der exprimierenden E.coli-Klone nach Induktion dargestellt (siehe Beispiel 1). Die Spuren entsprechen folgenden Ansätzen:
- Spur 1:: Expression des N-terminal verkürzten p18 (p18-30)
- Spur 2:: Expression des gesamt-p18 im pDS-Vektor
- Spur 3:: Expression des gesamt-p18 im pQE-Vektor.
- Spur 4:: Molekulargewichtsmarker

Die Größenunterschiede des gesamt-p18-Antigens beruhen auf unterschiedlichen N-terminalen Aminosäuren; der pQE-Vektor besitzt einen zusätzlichen Histidin-Strang.
Das N-terminal verkürzte p18 zeigt, wie erwartet ein ca. 4 kDa geringeres Molekulargewicht.

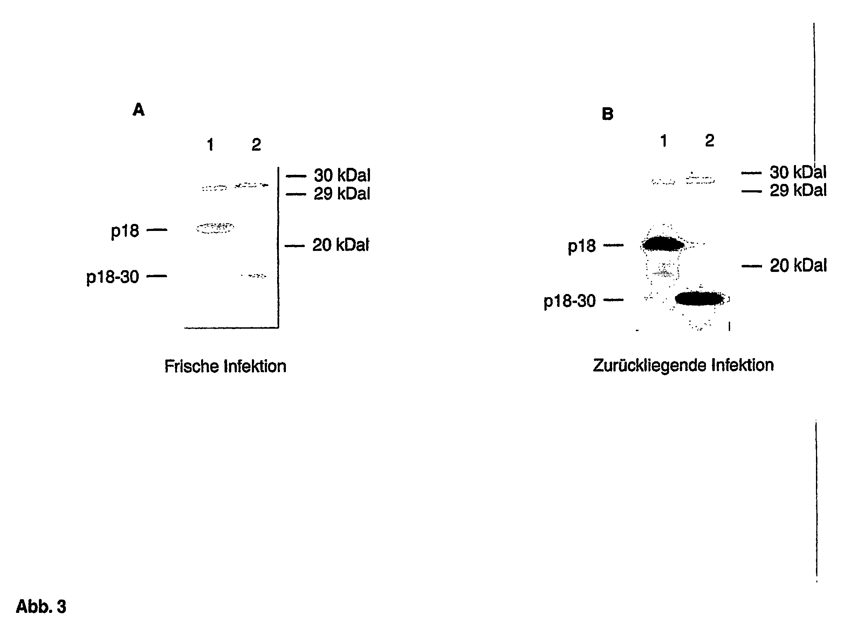

Figur 3 zeigt die Reaktivität der Peptide im Immunblot. Lysate von induzierten E.coli-Klonen mit den beiden p18-Varianten wurden Gel-elektrophoretisch aufgetrennt und anschließend auf Nitrozelluose-Membranen transferiert (Western Blot).
- Spur 1:: Gesamt p18 ohne His-tag
- Spur 2:: p18-30 (verkürzte p18-Version)

Die Nitrozellulose-Membranen wurden mit Seren EBV-positiver Personen inkubiert; spezifisch gebundene Antikörper wurden anschließend mit einem Peroxidase-konjugierten zweiten anti-human-IgG Antikörper and TMB-Färbung sichtbar gemacht.
A; Serum eines Patienten mit frischer EBV-Infektion
B; Serum mit lang zurückliegender EBV-Infektion

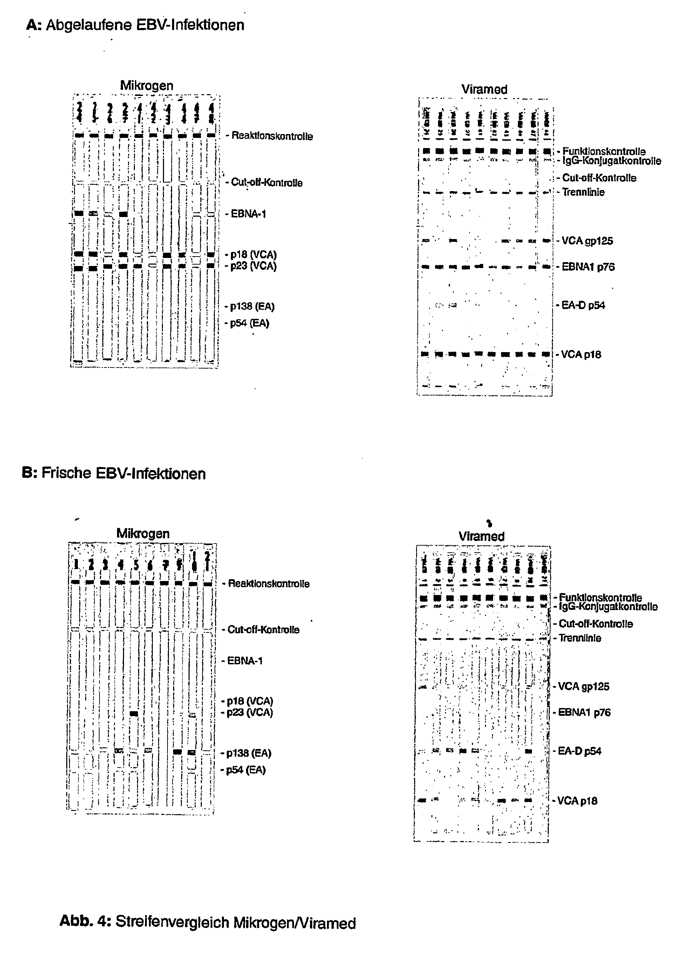

Figur 4 zeigt einen Reaktivitätsvergleich des N-terminal verkürzten p18 mit einem Volllängen-p18-VCA in einem kommerziell erhältlichen EBV-Antikörper-Test auf Streifen-Basis (Fa. Viramed, Planegg - ViraStripe EBV).

Jeweils 10 Seren von Personen mit lang zurückliegender EBV-Infektion, aber problematischer Serologie (schwache Antikörper-Titer, fehlendes anti-EBNA-1; A;) und 10 Proben von Patienten mit frischer EBV-Infektion (B;) wurden mit zwei Versionen von EBV-Antigen-Streifen getestet:
"Mikrogen"-Streifen enthalten neben dem N-terminal verkürzten p18 die zusätzlichen EBV-Antigene EBNA-1, p23 (VCA) sowie p54 und p138 (EAs).
Viramed-Streifen enthalten neben einem vollständigen p18 Antigen noch EBV-Proteine EBNA-1, EA-p54 sowie gp125 (VCA).

In der linken Hälfte (Mikrogen-Test) bezeichnet "p18" das N-terminal verkürzte p18. In der rechten Hälfte (Viramed-Test) bezeichnet "p18" das virale Volllängen-p18.

Bei den zurückliegenden Infektionen (A) ist bei beiden Tests immer eine mehr oder weniger ausgeprägte anti-p18 Reaktion zu finden.
Seren von Patienten nach Frischinfektion (B) zeigen in dem Test mit N-terminal verkürztem p18 jedoch in keinem Fall eine Reaktion, mit dem Virastripe (Gesamt-p18) jedoch in allen Proben.

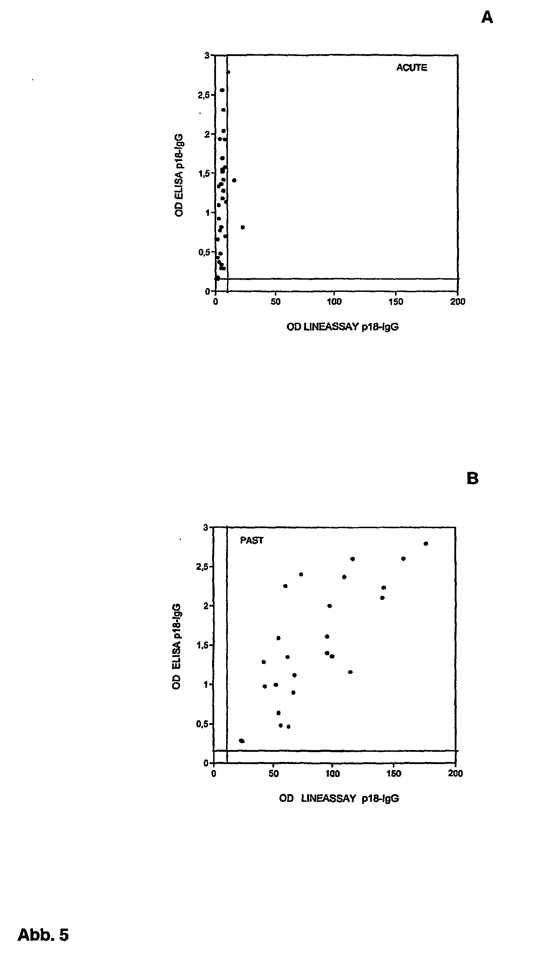

Figur 5 zeigt einen Reaktivitätsvergleich eines erfindungsgemäßen Tests mit einem kommerziell erhältlichen ELISA-System.

32 Proben von Patienten mit frischer EBV-Infektion ("acute") und 25 Serumproben von Personen mit abgelaufener (alter) EBV-Infektion ("past") wurden jeweils mit N-terminal verkürztem p18 (gesprüht auf Nitrozellulose-Streifen, Mikrogen, (vergleiche Antigenstreifen aus Abb. 4) und einem kommerziellen ELISA (Fa. Sorin) mit konventionellem p18-VCA-Antigen getestet.

In den beiden Diagrammen sind jeweils die OD's beider Teste gegeneinander aufgetragen; x-Achse - Intensität der Bande mit dem N-terminal verkürzten p18; Y-Achse - ELISA-Werte mit Gesamt-p18. Die OD-Werte des ELISA wurden durch mittels eines gängigen ELISA-Readers ermittelt und liegen zwischen 0 und 3,0; die Nitrozellulose-Streifen mit dem N-terminal verkürzten p18 wurden eingescannt und die Intensität der Bande mittels eines Software-Programms ermittelt; die Skala umfaßt hier Werte von 0 bis 200. Die beiden zusätzlichen Linien bei stellen die jeweiligen cut-off Grenzen dar.

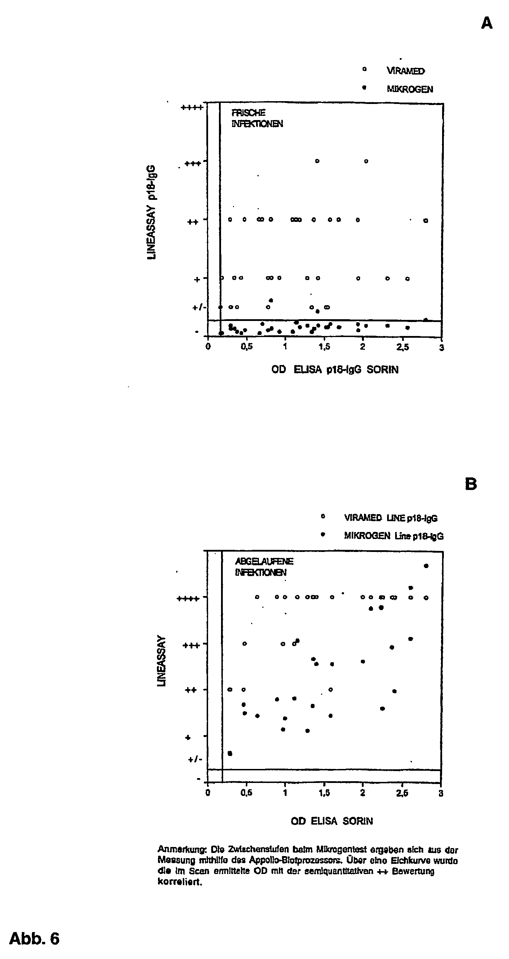

Figur 6 zeigt die Korrelation der p18-Reaktivität in verschiedenen Testformaten. Die Ergebnisse der Testung von Seren mit frischen und abgelaufenen Infektionen aus Beispiel 4 (Figur 5) wurden zusätzlich in Streifen-Tests mit konventionellem VCA-p18 (Fa. Viramed) untersucht und die Ergebnisse mit den VCA-ELISA-Werten und der Reaktivität mit dem N-terminal verkürzten p18 korreliert.

Die graphische Auswertung erfolgte ähnlich wie im vorigen Beispiel beschrieben: x-Achse mit den ELISA OD-Werten (von 0 - 3,0); y-Achse mit p18-Reaktivitäten der beiden Streifen-Teste (offene Kreise - Fa. Viramed mit Gesamt-p18; Punkte - N-terminal verkürztes p18 - Antigen).

Die nachfolgenden Beispiele erläutern die Erfindung näher.

### Beispiel 1: Rekombinante Darstellung von Gesamt-p18 und N-terminal verkürztem p18-Antigen

Als Beispiel für ein N-terminal verkürztes p18 wird die Expressionsklonierung eines Antigens beschrieben, dem gegenüber viralem Gesamt-p18 30 Aminosäuren N-terminal fehlen.

Der für die Aminosäuren 31-176 des p18-Antigens kodierende Bereich wurden mittels PCR und entsprechenden Oligonukleotid-Primern aus isolierter EBV-DNA (Stamm B95-8) amplifiziert.
(Leserahmen BFRF3; Sequenzdaten nach Genbank-Eintrag).

Primer für gesamt-p18:
5'-Primer:
GAG GGA TCC ATC ATG AAA **CGC CGG CTG CCC AAG CCC ACC** (SEQ ID NO:3)
3'-Primer:
CGC *CTG CAG* TTA **CTG TTT CTT ACG TGC CCC GCG** (SEQ ID NO:4)

Primer für verkürztes p18:
5'-Primer:
GAG *GGA TCC* **CTG AAC CAG AAT AAT CTC CCC** (SEQ ID NO:5)
3'-Primer:
CGC *CTG CAG* TTA **CTG TTT CTT ACG TGC CCC GCG** (SEQ ID NO:6)

Nukleotide in Fettschrift entsprechen p18-kodierenden Sequenzen; Nukleotide in Kursivschrift stellen Restriktionsenzymschnittstellen dar, die für weitere Klonierungsschritte verwendet wurden (GGATCC - BamHI; CTGCAG - Pst I).

Die PCR wurde in 100µl Ansätzen nach Standardmethoden und mit Standardpuffern durchgeführt (94°C Denaturierung 1 min; 55°C Primerbindung 1 min; 72°C Synthese 1 min; insgesamt 40 Zyklen).

Nach Überprüfung der PCR-Produkte auf richtige Größe in der Gel-Elektrophorese (ca. 530 nt für gesamt-p18 und ca. 440nt für verkürztes p18) wurden beide mittels konventioneller Methoden gereinigt und entsalzt, anschließend mit BamHl und Pstl geschnitten und schließlich in die entsprechenden Stellen des Expressionsvektors pDS1 eingesetzt.
pDS1 ist ein Vektor mit Ampicillin-Resistenz und einem optimierten lac-Promotor und anschließendem Translationsstart mit den BamHI und Pst I Stellen, die eine korrekte Translation der PCR-Produkte im richtigen Leserahmen vermitteln.
Andere Expressionssysteme sind auch geeignet.
Zu Vergleichszwecken wurde schließlich noch das gesamt-p18-kodierende Fragment in den kommerziellen Vektor pQE30 (QIAGEN) inseriert.

Nach Transformation wurden E.coli-Klone mit Expressionsplasmid und korrekten Inserts nach Standardmethoden induziert und die Lysate durch SDS-Gel-Elektrophorese und anschließende Coomassie-Färbung analysiert.

Beide Ansätze zeigen eine deutliche Expression von rekombinantem p18 bzw. p18-Derivat.
Das gesamt-p18 besitzt ein Molekulargewicht von ca. 22 kDa, das N-terminal um 30 Aminosäuren verkürzte Protein eine Größe von etwa 18 kDa mit einer etwas verringerten Expressionsausbeute.
Die pQE30-Expression des gesamt-p18 liegt in der Ausbeute in einem ähnlichen Bereich, aufgrund der zusätzlich vorhandenen Histidin-Reste ist das Produkt etwas größer (Fig. 2).

### Beispiel 2: Vergleich der Reaktivität von Gesamt-p18 mit N-terminal verkürztem Antigen.

Die Lysate der Klone mit gesamt- sowie verkürztem p18 wurden wiederum Gel-elektrophoretisch aufgetrennt, auf Nitrozellulose transferiert und anschließend auf ihre Reaktivität mit diversen EBV-positiven Seren untersucht.
Hierzu wurden die Nitrozellulose-Membranen mit 1:100 verdünnten Seren inkubiert und spezifische Antikörper mit einem zweiten Peroxidase-konjugierten Antikörper und anschließender Färbereaktion sichtbar gemacht.
Puffer, Verdünnungen und Inkubationszeiten wurden analog zu dem bei Mikrogen entwickelten EBV-Western Blot "recomBlot EBV" durchgeführt.
Andere Western Blot-Prozeduren oder Färbeverfahren können ebenfalls angewendet werden.

Gestestet wurde je ein Serum nach Frischinfektion (ca. 3 Wochen zurückliegend) sowie mit lange zurückliegender Infektion (ca. 1,5 Jahre zurückliegend) (Figur 3).
Überraschenderweise zeigt sich hier, daß das gesamt-p18-Antigen im Vergleich zu der verkürzten Version deutliche Reaktivitätsunterschiede aufweist:
Bei lange zurückliegender Infektion reagieren beide Antigenvarianten in etwa gleich stark; bei Seren von Frischinfektionen zeigt jedoch nur das gesamt-p18-Antigen eine deutliche Reaktion.

Damit ergibt sich ein zweiter Marker (neben EBNA-1), der für die zeitliche Einordnung einer EBV-Infektion verwendet werden kann.

### Beispiel 3: Reaktivitätsvergleich mit kommerziell erhältlichen Immundot-Tests

Die verkürzte Variante des p18-Antigens wurde in größeren Mengen exprimiert und mittels konventioneller Methoden aufgereinigt; die Schritte waren hierbei im Wesentlichen:
- Lyse der induzierten E.coli-Zellen mit dem Expressionsprodukt;
- Abtrennung von p18 als unlösliche "inclusion bodies" durch Zentrifugation;
- Waschen des unlöslichen Pellets mit diversen nicht-ionischen Detergenzien;
- Lösen des restlichen Pellets mit dem p18 in 8M Hamstoffpuffer;
- Verschiedene lonenaustausch-Chromatographien: DEAE, Q-Sepharose, S-Sepharose (alle Pharmacia).

Das Protein kann so in einer Reinheit >99% gewonnen werden.

Alternativ können auch andere Reinigungsverfahren oder Reihenfolgen von Säulenschritten verwendet werden. Bei Verwendung des pQE30-p18 Produkts kann insbesondere auch die selektive Anbindung an Ni-Chelat-Säulen ausgenutzt werden.

Das gereinigte Produkt wurde in einem "line assay" auf seine Reaktivität mit Antikörpern aus EBV-infizierten Individuen hin untersucht. Dazu wurde das Produkt zusammen mit anderen rekombinanten EBV-Antigenen (EBNA-1, p23-VCA, p138-EA, p54-EA) auf Nitrozellulose-Membranen mittels eines sogenannten Nadel-Linierers (isoflow, Fa. Imagene Inc.) in schmalen Linien aufgebracht und anschließend zu Teststreifen weiter prozessiert (Absättigung ungebundener Stellen auf der Membran, Aufbringen von Markierungen, Schneiden in schmale Streifen). Das Testprinzip des "line assay" entspricht dem kommerziell erhältlichen Immunassay "recomLine EBV IgG" der Firma Mikrogen (Artikel-Nr. 4572).

Die Streifen wurden mit Seren von EBV-Frischinfektionen und abgelaufenen Infektionen getestet. Dazu wurden die Streifen mit 1:100 verdünntem Serum inkubiert. Nach dreimaligem Waschen folgte eine Inkubation mit anti-human-IgG (Meerrettich-Peroxidase-Konjugat). Nach nochmaligem Waschen (dreifach) wurde mit TMB-Substratlösung inkubiert. Sobald die cut off-Kontrollbande zu sehen war, wurde die Substratlösung entfernt und die Streifen mit Wasser gewaschen. Eine Reaktion gilt als positiv, wenn die Intensität einer Bande gleich oder höher ist als die der cut off-Kontrollbande. Somit reagiert ein Peptid mit einem Antikörper, wenn eine Bandenintensität gleich dem "cut-off"-Wert oder oberhalb des "cut-off"-Werts erhalten wird. Keine oder eine schwache Reaktion bedeutet keine Bande bzw. eine Bande mit einer Intensität, die unterhalb des "cut off"-Werts liegt. Ein cut off-Wert in diesem Test ist dadurch definiert, daß die schwache Färbungsintensität einer sogenannten Kontrollbande auf dem Streifen ("cut off"-Bande) als Grenzwert für die Beurteilung der Reaktivität der Antigenbanden verwendet wird (siehe z.B. der oben genannte Immunassay "recomLine EBV IgG" der Firma Mikrogen). Die schwache Färbung der "cut off"-Bande ist durch die Verwendung bestimmter Reagenzien bei allen Durchführungen und Patientenproben immer gleich.

Als Vergleich wurde ein kommerzieller Streifentest der Fa. Viramed, Planegg (EBV-ViraStripe) durchgeführt, der sehr ähnlich den oben beschriebenen Streifen ist, aber zum Teil andere EBV-Antigene aufweist, darunter auch ein Gesamt-p18.

Wie aus der Figur 4 mit den immungefärbten Streifen deutlich hervorgeht, wird das Gesamt-p18 (rechts) bzw. das N-terminal verkürzte p18 (links) in beiden Tests von Seren mit abgelaufener EBV-Infektion gleich gut erkannt, die Reaktivitäten ähneln sich sehr. Deutlich wird der Unterschied hingegen bei den Seren frisch EBV-Infizierter:
Bei dem Vergleichstest mit herkömmlichem p18 wird dieses in allen Fällen mehr oder weniger stark erkannt; auf den Streifen mit dem verkürzten p18-Antigen ist in keinem der frischen Fälle eine Reaktion zu finden.

Dies unterstreicht wiederum die Wertigkeit und Zuverlässigkeit des veränderten p18 für eine Interpretation des EBV-Status.

### Beispiel 4: Reaktivitätsvergleich mit einem kommerziell erhältlichen ELISA-System

32 Proben von Patienten mit frischer EBV-Infektion ("acute") und 25 Serumproben von Personen mit abgelaufener (alter) EBV-Infektion ("past") wurden jeweils mit verkürztem p18 aus Beispiel 3 (gesprüht auf Nitrozellulose-Streifen, line assay p18) und einem kommerziellen ELISA (Epstein-Barr-Capsid-Antigen ETI-VCA-G; Fa. DiaSorin) mit konventionellem VCA-Antigen getestet (Figur 5).

In den beiden Diagrammen sind jeweils die OD's beider Tests gegeneinander aufgetragen. Die x-Achse entspricht der Intensität der Bande mit dem verkürzten p18. Die y-Achse entspricht den ELISA-Werten mit Gesamt-p18.
Die OD-Werte des ELISA wurden durch mittels eines gängigen ELISA-Readers ermittelt und liegen zwischen 0 und 3,0; die Nitrozellulose-Streifen mit dem verkürzten p18 wurden eingescannt und die Intensität der Bande mittels eines Software-Programms ermittelt; die Skala umfaßt hier Werte von 0 bis 200.
Die beiden zusätzlichen Linien bei stellen die jeweiligen cut-off Grenzen dar.

### Deutlich sind die Reaktivitätsunterschiede zu erkennen:

Bei den zurückliegenden Infektionen ähnelt sich die Reaktivitätsverteilung in hohem Maß - hoch positive Seren in dem einen Test werden vom anderen meist auch als stark positiv erkannt - d.h. bei zurückliegenden Infektionen reagieren das ELISA-Antigen und das verkürzte p18 sehr ähnlich.

Drastisch anders ist das Bild bei den Frischinfektionen: Bei den ELISA-Werten kommen - wie bei den zurückliegenden Infektionen auch - alle Werte zwischen cut-off-Grenze und
hoch positiv (OD 3,0) vor; es ist kein Unterschied zum Bild bei abgelaufenen Infektionen zu erkennen.
Die OD-Werte des verkürzten p18 liegen jedoch größtenteils unter der cut-off-Grenze und nur in zwei Fällen im schwach positiven Bereich. Dies demonstriert sehr eindrucksvoll die unterschiedliche Reaktivität des verkürzten p18 bei frischen und abgelaufenen Infektionen.

### Beispiel 5: Vergleichende Untersuchung der p18-Reaktivität in verschiedenen Testformaten

In einer weiteren Studie wurden die Seren von frischen und abgelaufenen Infektionen aus Beispiel 4 zusätzlich in Streifen-Testen mit konventionellen VCA-p18 (Fa. Viramed) untersucht und die Ergebnisse mit den VCA-ELISA-Werten und der Reaktivität mit dem verkürzten p18 aus Beispiel 3 korreliert (Figur 6).

### Die graphische Auswertung erfolgte ähnlich wie im vorigen Beispiel beschrieben:

Auf der x-Achse sind die ELISA OD-Werte aufgetragen (von 0 - 3,0); die y-Achse entspricht den p18-Reaktivitäten der beiden Streifen-Tests (offene Kreise - Gesamt-18 (Fa. Viramed), Punkte - verkürztes p18 -Antigen).

Bei dem Bild der Seren mit abgelaufener Infektionen sind - wie zu erwarten - alle Seren ELISA-positiv und gleichzeitig auch in beiden Streifen-Testen reaktiv; d.h. das Testformat hat keinen Einfluß.
Bei den Seren von frischen Infektionen zeigt sich jedoch wiederum die völlig andere Reaktivität des verkürzten p18: Die ELISA-Werte streuen - wie zu erwarten im Bereich des cut-off bis zu stark reaktiv; dasselbe Muster besitzen die Seren im Streifen-Test mit Gesamt-p18; das verkürzte p18-Antien hingegen ist im negativen oder - im Falle von zwei Proben - im schwach-positiven Bereich.

Diese Auswertung belegt nochmals sehr eindeutig die Unfähigkeit bisher verfügbarer EBV-Testsysteme zur Differenzierung von abgelaufener und frischer Infektion mit einem VCA-Marker. Durch die beschriebenen Maßnahmen kann ein verändertes (N-terminal verkürztes) p18-Antigen diese Unterscheidung leisten.

Damit ist bei Verwendung des verkürzten p18 in den allermeisten Fällen ein weiterer, sicherer Marker für die Diagnose einer Frischinfektion gefunden worden, der in Kombination mit den bekannten EBV-Markem eine wesentliche Verbesserung und Sicherheit bei der Interpretation es serologischen EBV-Status leisten kann.

### Weitere Literaturstellen:

G. Bauer. Die Aussagemöglichkeiten der Epstein-Barr-Virus-Diagnostik Der Internist 33: 586-592 (1992)
G. Bauer. Epstein-Barr-Virus: Bedeutung und Möglichkeiten der Labordiagnostik Therapeutische Umschau 51: 558-562 (1994),
G. Bauer. Diagnostik der Herpesviren. Teil I: Epstein-Barr-Virus (EBV) und Zytomegalievirus (CMV). mta 10, 502-508 (1995).
G. Bauer. Diagnostik der Herpesviren. Teil II: Herpes simplex-Virus (HSV), Varizella-Zoster-Virus (VZV) und Humanes Herpesvirus-6 (HHV-6). mta 10, 587-593 (1995)
G. Bauer. Rationale und rationelle EBV-Diagnostik. Clin. Lab. 41, 623 - 634 (1995).
G. Bauer. IgM - ein variabler und mehrdeutiger diagnostischer Marker. Der Mikrobiologe 6: 44-51 (1996)
T. Wolter, C. Gassmann, V. Vetter and G. Bauer. Avidity determination: utilization of a basic immunological mechanism allows to improve serological diagnosis of infections. Clin. Laboratory 43: 125-135, 1997.
M. Kampmann, K. Henninger and G. Bauer. Determination of antibodies directed specifically against Epstein-Barr virus nuclear antigen-1 (EBNA-1) by anticomplementary immunofluorescence (ACIF). Med. Microbiol. Letters 2: 1-8 (1993)
M. Schillinger, M. Kampmann, K. Henninger, G. Murray, I. Hanselmann, and G. Bauer. Variability of humoral immune response to acute Epstein-Barr virus (EBV) infection: evaluation of the significance of serological markers. Med. Microbiol. Letters 2: 296-303 (1993)
H.-J. Sohn and G. Bauer. Absorption of IgG increases the sensitivity of detection of the Epstein-Barr virus capsid antigen-specific lgM test. Med. Microbiol. Letters 2: 371-378 (1993)
V. Vetter, L. Kreutzer and G. Bauer. Differentiation of primary from secondary anti-EBNA-1-negative cases by determination of avidity of VCA-1gG. Clinical and Diagnostic Virology, 2: 29-39. (1994)
A. Andersson, V. Vetter, L. Kreutzer and G. Bauer. The avidities of IgG directed against viral capsid antigen (VCA) or early antigen (EA): useful markers for a more significant Epstein-Barr virus serology. J. Med. Virol., 43: 238-244 (1994)
G. Sigel, M. Schillinger, K. Henninger and G. Bauer. IgA directed against early antigen of Epstein-Barr virus is no specific marker for the diagnosis of nasopharyngeal carcinoma. J. Med. Virol., 43: 222-227 (1994)
W. Karner and G. Bauer. Activation of a varicella zoster virus-specific 1gA response during acute Epstein-Barr virus infection. J. Med. Virol., 44: 258-262 (1994).
C. Rapp, H. Berthold and G. Bauer: Loss of Anti-EBNA-1 during active hepatitis B virus infection: A diagnostic problem and indication for HBV-induced transient cellular immunosuppression. Med. Microbiol. Letters, 3: 235-243 (1994).
C. Alpers, C. Scheid and G. Bauer. Acute EBV infection results in IgM responses against unrelated viruses. Med. Microbiol. Letters 3: 306-314 (1994).

## Patentansprüche

1. Peptid, das vom viralen p18-Antigen des Epstein-Barr-Virus abgeleitet ist, und mit Antikörpern aus Individuen mit länger zurückliegender Epstein-Barr-Virus-Infektion reagiert, aber nicht oder schwach mit Antikörpern aus Individuen mit akuter oder kurz zurückliegender Epstein-Barr-Virus-Infektion, **dadurch gekennzeichnet, dass** es gegenüber dem viralen p18-Antigen N-terminal um 10 bis 70 Aminosäuren verkürzt ist.

2. Peptid nach Anspruch 1, **dadurch gekennzeichnet, dass** es die Aminosäuresequenz SEQ ID NO:2 aufweist.

3. Fusionspeptid umfassend ein Peptid nach einem der Ansprüche 1 oder 2 und eine oder mehrere weitere Aminosäuren, die nicht der Aminosäuresequenz des viralen p18-Antigens entsprechen.

4. Diagnostisches Reagens zum Nachweis von Antikörpern gegen Epstein-Barr-Virus umfassend ein Peptid nach einem der Ansprüche 1 oder 2 und/oder ein Fusionspeptid nach Anspruch 3.

5. Testkit zum Nachweis von Antikörpern gegen Epstein-Barr-Virus umfassend ein Peptid nach einem der Ansprüche 1 oder 2 und/oder ein Fusionspeptid nach Anspruch 3 und/oder ein diagnostisches Reagens nach Anspruch 4.

6. Verfahren zum Nachweis von Antikörpern gegen Epstein-Barr-Virus in einer Probe, das umfasst, dass man ein Peptid nach einem der Ansprüche 1 oder 2 oder ein Fusionspeptid nach Anspruch 3 oder ein diagnostisches Reagens nach Anspruch 4 mit der Probe in Kontakt bringt und anschließend die gebildeten Antigen-IgG-Antikörper-Komplexe nachweist.

7. Verfahren nach Anspruch 6, **dadurch gekennzeichnet, dass** wenigstens ein weiteres Antigen des EBV mit der Probe in Kontakt gebracht wird und anschließend die gebildeten Antigen-Antikörper-Komplexe nachgewiesen werden.

8. Verfahren nach Anspruch 7, **dadurch gekennzeichnet, dass** das weitere EBV-Antigen mit Antikörpern aus Individuen mit akuter oder kurz zurückliegender EBV-Infektion reagiert.

9. Verfahren nach Anspruch 8, **dadurch gekennzeichnet, dass** das weitere EBV-Antigen ausgewählt ist aus der Gruppe bestehend aus den viralen Antigenen des EBV p18, p23, p72, p138 und p54.

10. Verwendung eines modifizierten Viruskapsid-Antigens des Epstein-Barr-Virus zur Bestimmung in einer zu untersuchenden Probe, ob
a) eine akute oder kurz zurückliegende oder
b) eine abgelaufene oder länger zurückliegende EBV-Infektion vorliegt,
**dadurch gekennzeichnet, dass** das modifizierte Viruskapsid-Antigen ein Peptid nach einem der Ansprüche 1 oder 2 ist.

11. Vektor oder Plasmid enthaltend eine Nucleinsäure, die für ein Peptid nach einem der Ansprüche 1 oder 2 oder für ein Fusionspeptid nach Anspruch 3 codiert, **dadurch gekennzeichnet, dass** durch Expression in einer geeigneten Wirtszelle ein Peptid nach einem der Ansprüche 1 oder 2 oder ein Fusionspeptid nach Anspruch 3 erhältlich ist.

12. Zelle enthaltend einen Vektor oder ein Plasmid nach Anspruch 11.

## Claims

1. A peptide which is derived from the viral p18 antigen of the Epstein-Barr virus and which reacts with antibodies from individuals having an Epstein-Barr virus infection dating back to the relatively distant past, but not or weakly with antibodies from individuals having an acute Epstein-Barr virus infection or an Epstein-Barr virus infection dating back to the recent past, **characterized in that** it is shortened at the N-terminus by 10 to 70 amino acids compared with the viral p18 antigen.

2. A peptide as claimed in claim 1 having the amino acid sequence SEQ ID NO:2.

3. A fusion peptide comprising a peptide as claimed in any of claims 1 or 2 and one or more further amino acids which do not correspond to the amino acid sequence of the viral p18 antigen.

4. A diagnostic reagent for the detection of antibodies against Epstein-Barr virus, comprising a peptide as claimed in any of claims 1 or 2 and/or a fusion peptide as claimed in claim 3.

5. A test kit for the detection of antibodies against Epstein-Barr virus, comprising a peptide as claimed in any of claims 1 or 2 and/or a fusion peptide as claimed in claim 3 and/or a diagnostic reagent as claimed in claim 4.

6. A method for the detection of antibodies against Epstein-Barr virus in a sample, which comprises bringing a peptide as claimed in any of claims 1 or 2 or a fusion peptide as claimed in claim 3 or a diagnostic reagent as claimed in claim 4 into contact with the sample and then identifying the antigen-IgG-antibody complexes formed.

7. The method as claimed in claim 6, wherein at least one further antigen of EBV is brought into contact with the sample and the antigen-antibody complexes formed are then identified.

8. The method as claimed in claim 7, wherein the further EBV antigen reacts with antibodies from individuals having an acute EBV infection or an EBV infection dating back to the recent past.

9. The method as claimed in claim 8, wherein the further EBV antigen is selected from the group consisting of the viral antigens of EBV p18, p23, p72, p138 and p54.

10. The use of a modified virus capsid antigen of the Epstein-Barr virus for determining in a sample to be tested whether
a) an acute EBV infection or EBV infection dating back to the recent past or
b) a past EBV infection or an EBV infection dating back to the relatively distant past is present,
wherein the modified virus capsid antigen is a peptide as claimed in any of claims 1 or 2.

11. A vector or plasmid comprising a nucleic acid which codes for a peptide as claimed in any of claims 1 or 2 or for a fusion peptide as claimed in claim 3, wherein a peptide as claimed in any of claims 1 or 2 or a fusion peptide as claimed in claim 3 is obtainable by expression in a suitable host cell.

12. A cell comprising a vector or a plasmid as claimed in claim 11.

## Revendications

1. Peptide dérivé de l'antigène p18 du virus Epstein-Barr et qui réagit avec des anticorps d'individus ayant une infection au virus Epstein-Barr ancienne, mais pas ou faiblement avec des anticorps d'individus ayant une infection aiguë ou récente au virus Epstein-Barr, **caractérisé en ce qu'**il est raccourci de 10 à 70 acides aminés N-terminaux par rapport à l'antigène viral p18 .

2. Peptide selon la revendication 1 **caractérisé en ce qu'**il présente la séquence d'acides aminés SEQ ID NO : 2.

3. Peptide de fusion comprenant un peptide selon la revendication 1 ou 2 et un ou plusieurs autres acides aminés qui ne correspondent pas à la séquence d'acides aminés de l'antigène viral p18.

4. Réactif de diagnostic pour la caractérisation d'anticorps contre le virus Epstein-Barr comprenant un peptide selon une des revendications 1 ou 2 et/ou un peptide de fusion selon la revendication 3.

5. Nécessaire de test pour la caractérisation d'anticorps contre le virus Epstein-Barr comprenant un peptide selon une des revendications 1 ou 2 et/ou un peptide de fusion selon la revendication 3 et/ou un réactif de diagnostic selon la revendication 4.

6. Procédé de caractérisation d'anticorps contre le virus Epstein-Barr dans un échantillon qui inclut que l'on mette en contact un peptide selon une des revendications 1 ou 2 ou un peptide de fusion selon la revendication 3 ou un réactif de diagnostic selon la revendication 4 avec l'échantillon puis que l'on caractérise les complexes antigène-IgG-anticorps formés.

7. Procédé selon la revendication 6 **caractérisé en ce qu'**au moins un autre antigène du virus Epstein-Barr est mis en contact avec l'échantillon puis que l'on caractérise les complexes antigène-IgG-anticorps formés.

8. Procédé selon la revendication 7 **caractérisé en ce que** l'autre antigène du virus Epstein-Barr réagit avec des anticorps d'individus ayant une infection aiguë ou récente au virus Epstein-Barr.

9. Procédé selon la revendication 8 **caractérisé en ce que** l'autre antigène du virus Epstein-Barr est choisi dans le groupe constitué des antigènes viraux des virus Epstein-Barr p18, p23, p72, p138 et p54.

10. Utilisation d'un antigène de capside virale modifié du virus Epstein-Barr pour déterminer dans un échantillon à étudier la présence :
- d'une infection au virus Epstein-Barr aiguë ou récente, ou
- d'une infection au virus Epstein-Barr terminée ou ancienne,
**caractérisée en ce que** l'antigène de capside virale modifié est un peptide selon une des revendications 1 ou 2.

11. Vecteur ou plasmide contenant un acide nucléique qui code un peptide selon une des revendications 1 ou 2 ou un peptide de fusion selon la revendication 3, **caractérisé en ce qu'**on peut obtenir un peptide selon une des revendications 1 ou 2 ou un peptide de fusion selon la revendication 3 par expression dans une cellule hôte appropriée.

12. Cellule contenant un vecteur ou un plasmide selon la revendication 11.


### SEQUENZPROTOKOLL

<110> MIKROGEN molekularbiologische Entwicklungs-GmbH
<120> Von Kapsidantigenen des Epstein-Barr-Virus abgeleitete Peptide und ihre Verwendung
<130> modifiziertes VCA
<160> 6
<170> PatentIn version 3.1
<210> 1
   <211> 176
   <212> PRT
   <213> Human herpesvirus 4
<400> 1
   Met Ala Arg Arg Leu Pro Lys Pro Thr Leu Gln Gly Arg Leu Glu Ala
   1 5 10 15
   Asp Phe Pro Asp Ser Pro Leu Leu Pro Lys Phe Gln Glu Leu Asn Gln
   20 25 30
   Asn Asn Leu Pro Asn Asp Val Phe Arg Glu Ala Gln Arg Ser Tyr Leu
   35 40 45
   Val Phe Leu Thr Ser Gln Phe Cys Tyr Glu Glu Tyr Val Gln Arg Thr
   50 55 60
   Phe Gly Val Pro Arg Arg Gln Arg Ala Ile Asp Lys Arg Gln Arg Ala
   65 70 75 80
   Ser Val Ala Gly Ala Gly Ala His Ala His Leu Gly Gly Ser Ser Ala
   85 90 95
   Thr Pro Val Gln Gln Ala Gln Ala Ala Ala Ser Ala Gly Thr Gly Ala
   100 105 110
   Leu Ala Ser Ser Ala Pro Ser Thr Ala Val Ala Gln Ser Ala Thr Pro
   115 120 125
   Ser Val Ser Ser Ser Ile Ser Ser Leu Arg Ala Ala Thr Ser Gly Ala
   130 135 140
   Thr Ala Ala Ala Ser Ala Ala Ala Ala Val Asp Thr Gly Ser Gly Gly
   145 150 155 160
   Gly Gly Gln Pro His Asp Thr Ala Pro Arg Gly Ala Arg Lys Lys Gln
   165 170 175
<210> 2
   <211> 146
   <212> PRT
   <213> Künstliche Sequenz
<220>
   <223> N-terminal verkürztes p18
<400> 2
   Asn Gln Asn Asn Leu Pro Asn Asp Val Phe Arg Glu Ala Gln Arg Ser
   1               5                   10                  15
   Tyr Leu Val Phe Leu Thr Ser Gln Phe Cys Tyr Glu Glu Tyr Val Gln
               20                  25                  30
   Arg Thr Phe Gly Val Pro Arg Arg Gln Arg Ala Ile Asp Lys Arg Gln
           35                  40                  45
   Arg Ala Ser Val Ala Gly Ala Gly Ala His Ala His Leu Gly Gly Ser
       50                  55                  60
   Ser Ala Thr Pro Val Gln Gln Ala Gln Ala Ala Ala Ser Ala Gly Thr
   65                  70                  75                  80
   Gly Ala Leu Ala Ser Ser Ala Pro Ser Thr Ala Val Ala Gln Ser Ala
                   85                  90                  95
   Thr Pro Ser Val Ser Ser Ser Ile Ser Ser Leu Arg Ala Ala Thr Ser
               100                 105                 110
   Gly Ala Thr Ala Ala Ala Ser Ala Ala Ala Ala Val Asp Thr Gly Ser
           115                 120                 125
   Gly Gly Gly Gly Gln Pro His Asp Thr Ala Pro Arg Gly Ala Arg Lys
       130                 135                 140
   Lys Gln
   145
<210> 3
   <211> 39
   <212> DNA
   <213> Künstliche Sequenz
<220>
   <223> Primer
<400> 3
   gagggatcca tcatgaaacg ccggctgccc aagcccacc 39
<210> 4
   <211> 33
   <212> DNA
   <213> Künstliche Sequenz
<220>
   <223> Primer
<400> 4
   cgcctgcagt tactgtttct tacgtgcccc gcg 33
<210> 5
   <211> 30
   <212> DNA
   <213> Künstliche Sequenz
<220>
   <223> Primer
<400> 5
   gagggatccc tgaaccagaa taatctcccc 30
<210> 6
   <211> 33
   <212> DNA
   <213> Künstliche Sequenz
<220>
   <223> Primer
<400> 6
   cgcctgcagt tactgtttct tacgtgcccc gcg 33